# EUROPEAN PATENT APPLICATION

(11) **EP 3 566 573 A1**
(43) Date of publication of application: **13.11.2019**
(21) Application number: 19172914.4
(22) Date of filing: 07.05.2019
(51) Int. Cl.: A01K 63/04

(54) **SYSTEM AND METHOD FOR AUTOMATIC WATER QUALITY ADJUSTMENT**

(30) Priority: 08.05.2018 US 201862668431 P
(71) Applicant: NATIONAL CHUNG CHENG UNIVERSITY, Chiayi County 621 (TW); SANDASTER INTERNATIONAL LTD., Tainan City 700 (TW)
(72) Inventor: TING, Chu-Chi, 621 Chiayi County (TW); HUANG, Yu-Wen, 700 Tainan City (TW)
(74) Representative: Michalski Hüttermann & Partner Patentanwälte mbB

(57) **Abstract**

An automatic water quality adjustment system (1000) and an automatic water quality adjustment method are provided. The automatic water quality adjustment system (1000) **comprises** a reaction tank (1), a sample dripping device (2), a titrant dripping device (5), a color sensor (6), a first light interception counter (107, 7), a waste removal device (8), a control unit (9), and a supplementary device (10). The automatic water quality adjustment system (1000) quantifies a content of a specific element in an aqueous solution by titration. The automatic water quality adjustment system (1000) will add a supplement (1021, 1023) into the aqueous solution in order to stabilize the content of the specific element in the aqueous solution.

## Description

### BACKGROUND OF THE INVENTION

### 1. Field of the Invention

The present invention relates to a system and a method for automatic water quality adjustment, particularly, to a system and a method for automatically adjusting the water quality of an aquarium fish tank.

### 2. Description of Related Art

The general aquarium ornamental fish tank and farmed fish tank are semi-closed systems, the content of minerals and organic substance in the water, pH value, and hardness of the water of the aquarium fish tank are easily changed due to the physiological effects of fishes or other organisms living in the fish tank; therefore, the deterioration of water quality may cause illness or death of those organisms in the fish tank. Particularly, the water quality may change significantly when adding water to the fish tank or changing the water of the fish tank, thus the water quality should be monitored and adjusted to ensure the healthiness and comfort of the organisms in the fish tank.

For instance, the content of salt in the water and the hardness of the water are critical factors for breeding marine fishes which must be in a specific range that suitable for the growth of the marine fishes. If corals or aquatic plants are also kept in the fish tank, the hardness of the water may decrease due to the coral growth. The osmotic pressure and pH value of water may further be influenced. Accordingly, the fishes and other creatures may feel discomfort, become sick, or be dead while their living environment of changes. To avoid the water quality change in the fish tank, supplements are generally added into the fish tank for maintaining the healthy life to those aquarium organisms.

However, the supplements for the aquarium fish tank are added manually. In general, the content of one specific factor must be tested using a test strip of a sensor; then calculate the amount of the supplement to be added according to the volume of the water in the fish tank. The process is complicated and time-consuming. The water quality might become worse and cause illness to the aquarium organisms if too much or too fewer supplements were added due to estimation errors.

Therefore, an automatic water quality adjustment system is required to automatically monitor the water quality and adjusting the water quality of the ornamental fish tanks, farmed fish tanks, or fish ponds.

### SUMMARY OF THE INVENTION

To achieve the above-mentioned object, the present invention provides an automatic water quality adjusting system. The system utilizes the titration method to determine the mineral contents, the organic substance contents, pH value, and hardness, etc of a water solution, and calculates the dosage of supplements that are added to the water solution afterwards so that the water quality parameters (such as mineral contents, organic substance contents, pH value, and hardness) may be maintained at a predetermined value.

The automatic water quality adjusting system mainly comprises: a reaction tank including an opening; a sample dripping device including a sample suction tube and a sample dropper, wherein a dropper tip of the sample dropper extends to the opening of the reaction tank and interconnects with the sample suction tube, so that a test solution, which is a portion of the liquid drawn by the sample suction tube, is dropped into the reaction tank; at least one titrant dripping device including a titrant dropper, wherein a dropper tip of the titrant dropper extends to the opening of the reaction tank so that a titrant is dropped into the reaction tank for titrating the test solution in the reaction tank; a color sensor disposed on a side of the reaction tank for detecting the color change of the test solution when reacting with the titrant; a first light interception counter disposed adjacent to the opening for counting number of drops of the test solution and the titrant dropped into the reaction tank; a waste removal device including a waste suction tube and a waste discharge tube, one end of the waste suction tube interconnects with the reaction tank, another end of the waste suction tube interconnects with the waste discharge tube, wherein the waste discharge tube draws the waste liquid generated after completion of the titration in the reaction tank; a control unit controlling drop frequency and number of drops of the test solution and the titrant, discharging waste of the waste removal device, and receiving and processing signals from the color sensor and the first light interception counter; a supplementary device including at least one supplement output tube, one end of the at least one supplement output tube outputs at least one supplement to the liquid. The at least one titrant dripping device titrates the test solution in the reaction tank with the titrant; the first light interception counter counts the number of drops of the titrant; the color sensor detects the color change of the test solution and reacting with the titrant to determine whether the titration is complete; and the control unit calculates a titer of the titrant detected by the first light interception counter and processes the titer of the titrant into an output amount of the supplement; then, the supplement of the output amount is outputted to the liquid.

According to a preferred embodiment of the present invention, the at least one titrant dripping device further includes a titrant suction tube, a titrant storage tank, and a titrant pump, wherein the titrant is stored in the titrant storage tank, one end of the titrant suction tube extends to the titrant storage tank, another end of the titrant suction tube connects with the titrant pump and a tube body of the titrant dropper also connects with the titrant pump. The titrant pump drives the titrant suction tube to draw the titrant from the titrant storage tank and drives the titrant dropper to drop the titrant into the reaction tank.

According to a preferred embodiment of the present invention, the automatic water quality adjusting system further comprises at least one indicator dripping device, wherein the at least one indicator dripping device includes an indicator dropper, a dropper tip of the indicator dropper extends to the opening of the reaction tank so that an indicator is dropped into the reaction tank for reacting with the test solution and rendering color. In a more preferred embodiment, the at least one indicator dripping device further includes an indicator suction tube, an indicator storage tank, and an indicator pump, wherein the indicator is stored in the indicator storage tank, one end of the indicator suction tube extends to the indicator storage tank, another end of the indicator suction tube connects with the indicator pump, and a tube body of the indicator dropper also connects with the indicator pump. The indicator pump drives the indicator suction tube to draw the indicator from the indicator storage tank and drives the indicator dropper to drop the indicator into the reaction tank. The number of drops of the indicator, which represents the amount of the indicator, is counted by the first light interception counter.

According to a preferred embodiment of the present invention, the automatic water quality adjustment system further comprises at least one precipitant dripping device, wherein the at least one precipitant dripping device includes a precipitant dropper, a dropper tip of the precipitant dropper extends to the opening of the reaction tank so that a precipitant is dropped into the reaction tank for precipitating impurities of the test solution in the reaction tank. In a more preferred embodiment, the at least one precipitant dripping device further includes a precipitant suction tube, a precipitant storage tank, and a precipitant pump, wherein the precipitant is stored in the precipitant storage tank, one end of the precipitant suction tube extends to the precipitant storage tank, another end of the precipitant suction tube connects with the precipitant pump, and a tube body of the precipitant dropper also connects with the precipitant pump. The precipitant pump drives the precipitant suction tube to draw the precipitant from the precipitant storage tank and drives the precipitant dropper to drop the precipitant into the reaction tank. The number of drops of the precipitant, which represents the amount of the precipitant, is counted by the first light interception counter.

According to a preferred embodiment of the present invention, the sample dripping device further includes a sample pump, wherein the one end of the sample suction tube extends into the liquid, another end of the sample suction tube connects with the sample pump, and a tube body of the sample dropper also connects with the sample pump. The sample pump drives the sample suction tube to draw the portion of the liquid as the test sample and drives the sample dropper to drop the test sample into the reaction tank.

According to a preferred embodiment of the present invention, the waste removal device further includes a waste pump, wherein another end of the waste suction tube connects with the waste pump. The waste pump drives the waste suction tube to draw the waste from the reaction tank and discharges the waste from the waste discharge tube.

According to a preferred embodiment of the present invention, the supplementary device further includes at least one supplementary suction tube, at least one supplement storage tank, at least one supplementary pump, a supplement tank, and a second supplementary pump, wherein one end of the at least one supplementary suction tube extends to the at least one supplement storage tank, a dropper tip at another end of the at least one supplementary suction tube extends to an opening of the supplement tank, at least one supplement is stored in the at least one supplement storage tank respectively, and the supplementary output tube interconnects with the supplement tank, wherein the at least one first supplementary pump drives the at least one supplementary suction tube to draw the at least one supplement from the at least one supplement storage tank and drop the at least one supplement into the supplement tank, then the second supplementary pump drives the supplementary output tube to output the at least one supplement into the liquid.

According to a preferred embodiment of the present invention, the supplementary device further includes a second light interception counter disposed at the opening of the supplement tank for counting the number of drops of the at least one supplement that dropped into the supplement tank.

According to a preferred embodiment of the present invention, the automatic water quality adjustment system further includes a stirrer disposed in the reaction tank for uniformly mixing the test solution and at least one selected from the group consisting of the precipitant, the indicator, and the titrant. In a more preferred embodiment, the stirrer includes a stir bar and a magnet mixing motor, wherein the stir bar is disposed in the reaction tank, and the magnet mixing motor is disposed at an outer side of the reaction tank for driving the stir bar.

The present invention provide an automatic water quality adjustment method using the automatic water quality adjustment device that described above, wherein the method comprises the steps of: Step (1) using the sample dripping device to draw a portion of the liquid as the test solution and to drop the test solution into the reaction tank, and using the first light interception counter to count the number of drops of the test solution for calculating a volume of the test solution; Step (2): using the at least one titrant dripping device to drop the titrant into the reaction tank to react with the test solution for performing a titration, using the first light interception counter to record the number of drops of the titrant, and using the color sensor to detect the color change while titration, wherein the titration is completed when the color of the test solution reaches to a pre-determined color; and Step (3): using the control unit to calculate the titer of the titrant detected by the first interception counter and to process the titer of the titrant into the output amount of the supplement, then the supplement with the output amount is outputted to the liquid by the supplementary device.

According to a preferred embodiment of the present invention, the automatic water quality adjustment method further comprises a Step (1-1): using the at least one precipitant dripping device to drop the precipitant into the reaction tank for reacting the precipitant and the test solution.

According to a preferred embodiment of the present invention, the automatic water quality adjustment method further comprises a Step (1-2): using the at least one indicator dripping device to drop the indicator into the reaction tank for reacting the indicator and the test solution.

According to a preferred embodiment of the present invention, the automatic water quality adjustment method further comprises a Step (2-1): using the waste removal device to discharge the waste in the reaction tank after the Step (2).

According to the automatic water quality adjustment system of the present invention, the content of calcium ion, magnesium ion, barium ion, iron ion, aluminum ion, silicon, potassium ion, copper ion, manganese ion, fluoride ion, chloride ion, bromide ion, iodide ion, nitrate ion, nitrite ion, phosphate ion, amine, ammonium ion, and the hardness of water in the liquid is adjustable. According to the specific elements to be adjusted, the automatic water quality adjustment device may comprise a plurality of precipitant dripping device, a plurality of indicator dripping device, a plurality of titrant dripping device, and a plurality of supplementary device, wherein the number of these devices may be altered according to the needs without limitation. The species of the precipitants, the indicators, the titrants, and the supplements are determined according to the elements to be adjusted and is known in the art. Therefore, a person skilled in the art can use a commercially available pharmaceutical agent as the precipitants, the indicators, the titrants, and the supplement, or can be prepared by those skilled in the art without limitations.

### BRIEF DESCRIPTION OF THE DRAWINGS

FIG. 1 is an appearance perspective view of the automatic water adjustment system of an embodiment of the present invention;
FIG. 2 is a perspective view of the automatic water adjustment system of an embodiment of the present invention;
FIG. 3 is a perspective view of the automatic water adjustment system of an embodiment of the present invention; and
FIG. 4 is a schematic plan view of the automatic perspective view of the automatic water adjustment system of an embodiment of the present invention.

### DETAILED DESCRIPTION OF THE PREFERRED EMBODIMENT

Hereafter, examples will be provided to illustrate the embodiments of the present invention. Advantages and effects of the invention will become more apparent from the disclosure of the present invention. It should be noted that these accompanying figures are simplified and illustrative. The quantity, shape, and size of components shown in the figures may be modified according to practical conditions, and the arrangement of components may be more complex. Other various aspects also may be practiced or applied in the invention, and various modifications and variations can be made without departing from the invention based on various concepts and applications.

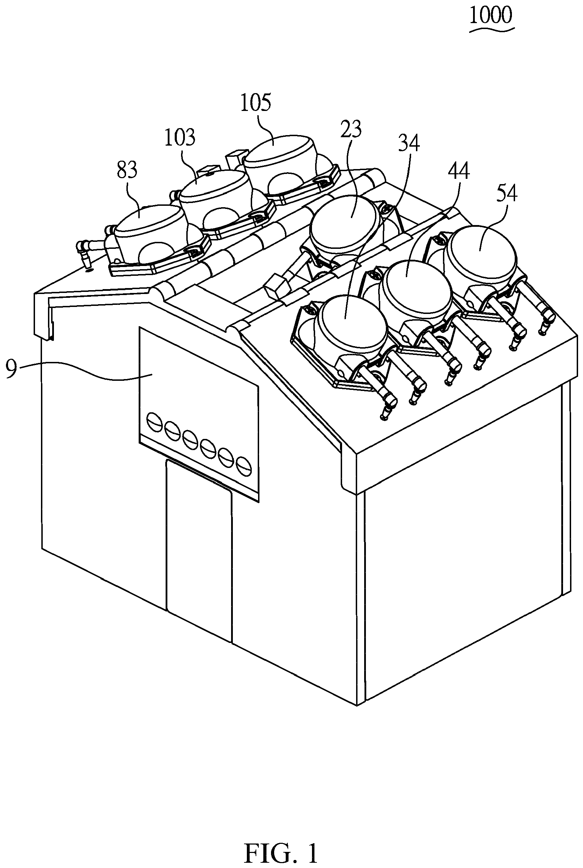

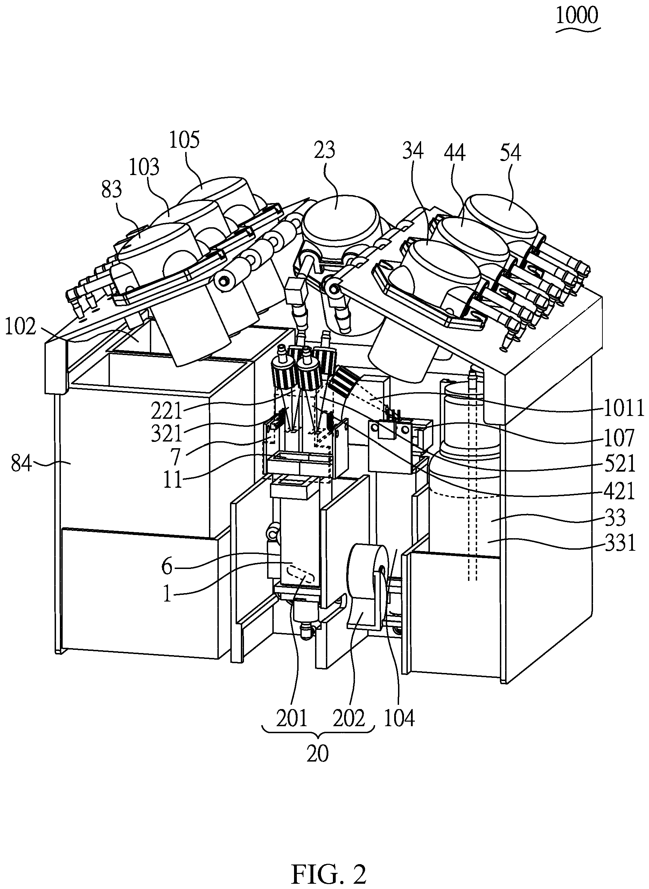

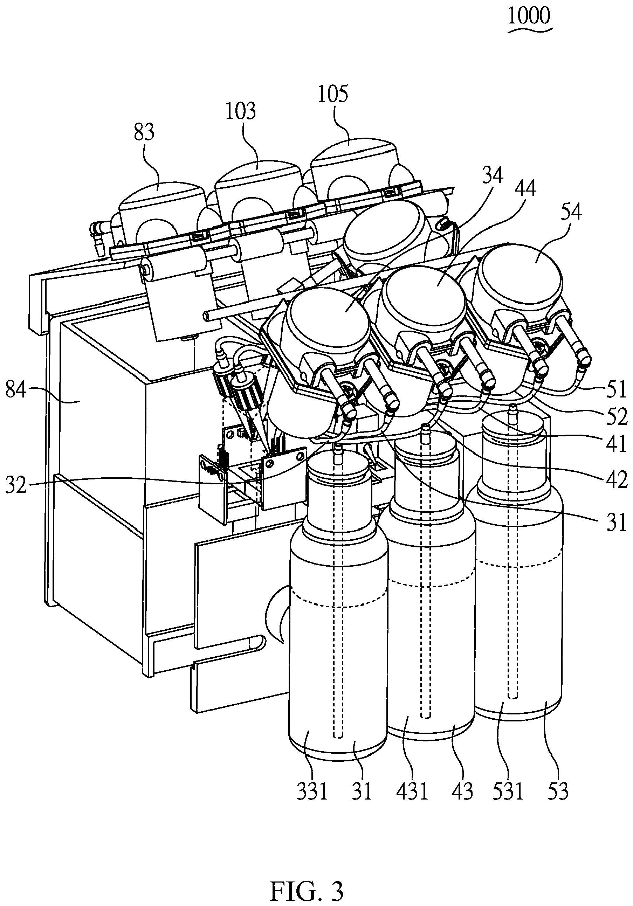

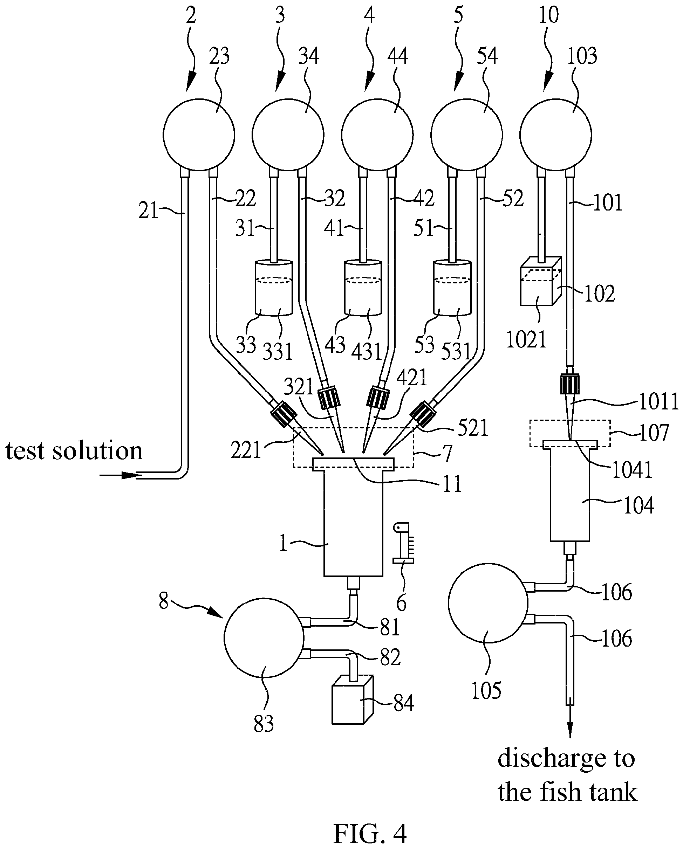

Please refer to FIG. 1 to FIG. 4 wherein FIG. 1 illustrates the appearance of the automatic water quality adjustment system 1000; FIG. 2 and FIG. 3 illustrate the perspective view of the automatic water quality adjustment system 1000, and FIG. 4 illustrates the schematic plan view of the automatic water quality adjustment system 1000 of the present embodiment. In order to clearly mark each of the elements and keep the drawings simple, the pipelines (including suction tubes, droppers, and discharge tubes) of the automatic water quality adjustment system 1000 are omitted in FIG. 2 and FIG. 3. However, the connections of those pipelines are clearly illustrated in FIG. 4. Therefore, please refer to FIG. 2 to FIG. 4 simultaneously to fully understand the configuration of the automatic water quality adjustment system. In addition, the automatic water quality adjustment system 1000 of the present embodiment is exemplified by adjusting the content of calcium ions in a seawater aquarium fish tank. In order to quantify the calcium ion in the seawater aquarium fish tank, the precipitant, indicator, and titrant used in the present embodiment are solutions for titrating calcium ions known in the art.

The automatic water quality adjustment system 1000 of the present embodiment comprises a reaction tank 1; a sample dripping device 2; a precipitant dripping device 3; an indicator dripping device 4; a titrant dripping device 5; a color sensor 6; a first light interception counter 7; a water removal device; a control unit 9; a supplementary device; and a stirrer 20.

Specifically, the reaction tank 1 is a transparent acrylic reaction tank including an opening 11 facing upwards; the sample dripping device 2 includes a sample suction tube 21, a sample dropper 22, and a sample pump 23, wherein the sample suction tube 21 interconnects to the sample dropper 22. The sample dropper 22 has a dropper tip 221 that extends to the opening 11 of the reaction tank 1. The sample suction tube 21 draws a portion of the water in the aquarium fish tank as the test solution. The sample pump 23 is a peristaltic pump and drives the sample suction tube 21 to draw the test solution and deliver the test solution to the sample dropper 22. The test solution was dropped into the reaction tank 1 through the dropper tip 221 of the sample dropper 22.

The precipitant dripping device 3 includes a precipitant suction tube 31, a precipitant dropper 32, a precipitant storage tank 33, and a precipitant pump 34, wherein one end of the precipitant suction tube 31 extends to the precipitant storage tank 33 and another end of the precipitant suction tube 31 interconnects with the precipitant dropper 32. The precipitant dropper 32 has a dropper tip 321 that extends to the opening 11 of the reaction tank 1. The precipitant pump 34 drives the precipitant suction tube 31 to draw a precipitant 331 that stored in the precipitant storage tank 33 and to deliver the precipitant 331 to the precipitant dropper 32. The precipitant 331 is then dropped to the reaction tank 1 through the dropper tip 321 of the precipitant dropper 32 and reacts with the test solution. In the present embodiment, the precipitant 331 is added to the test solution for precipitating impurities of the test solution and increasing the accuracy of the subsequent titration. In other embodiment, the precipitant 331 may be added or may be omitted according to the requirements of the titration.

The indicator dripping device 4 includes an indicator suction tube 41, an indicator dropper 42, an indicator storage tank 43, and an indicator pump 44. One end of the indicator suction tube 41 extends to the indicator storage tank 43, another end of the indicator suction tube 41 interconnects with the indicator dropper 52. The indicator dropper 42 has a dropper tip 421 that extends to the opening 11 of the reaction tank 1. An indicator 431 is stored in the indicator storage tank 43. The indicator pump 44 drives the indicator suction tube 41 to draw the indicator 431 and to deliver the indicator 431 to the indicator dropper 42. The indicator 431 is then dropped to the reaction tank 1 through the dropper tip 421 of the indicator dropper 42 and reacts with the test solution.

The titrant dripping device 5 includes a titrant suction tube 51, a titrant dropper 52, a titrant storage tank 53, and a titrant pump 54. One end of the titrant suction tube 51 extends to the titrant storage tank 53, another end of the titrant suction tube 51 interconnects with the titrant dropper 52. The titrant dropper 52 has a dropper tip 521 that extends to the opening 11 of the reaction tank 1. A titrant 531 is stored in the titrant storage tank 53. The titrant pump 54 drives the titrant suction tube 51 to draw the titrant 531 form the titrant storage tank 53 and to deliver the titrant 531 to the titrant dropper 52. The titrant 531is then dropped to the reaction tank 1 through the dropper tip 521 of the indicator dropper 52 for titration of the test solution.

The stirrer 20 includes a stir bar 201 and a magnet mixing motor 202. The stir bar 201 is disposed in the reaction tank 1 and the magnet mixing motor 202 is disposed at an outer side of the reaction tank 1. The stirrer 20 is used to uniformly mix the test solution and the precipitant 331, the indicator 431, and the titrant 531. The stirrer 20 can uniformly mix the test solution and the titrant so that the color change due to the titration may be accurately detected by the color sensor 6.

The color sensor 6 is disposed at one side of the reaction tank 1 and aiming the reaction tank 1. When the titration begins, the color sensor 6 detects the color of the test solution in the reaction tank 1 three seconds after dropping one drop of titrant 531 by the titrant dripping device 5 for obtaining a color parameter. The titration end point is reached when the color parameter reaches a predetermined value.

The first light interception counter 7 is disposed at the opening 11 of the reaction tank 1 for recording the number of drops of the precipitant 331, the indicator 431, and the titrant 531 so that the volume of the precipitant 331, the indicator 431, and the titrant 531 that dropped into the reaction tank 1 may be calculated.

The waste removal device 8 includes a waste suction tube 81, a waste discharge tube 82, a waste pump 83, and a waste storage tank 84. One end of the waste suction tube 81 interconnects with the reaction tank 1, another end of the waste suction tube 81 interconnects with the wasted discharge tube 82. When the titration is over, the waste pump 83 drives the waste suction tube 82 to draw the waste from the reaction tank 1 and discharge the wasted through the waste discharge tube 82 from the automatic water quality adjustment system 1000.

The control unit 9 controls the drop frequency of the sample dripping device 2, the precipitant dripping device 3, the indicator dripping device 4, and the titrant dripping device 5; receives the signals of the number of drops that detected by the first light interception counter 7 and processes those signals to obtain the volume of the test solution, the precipitant, the indicator, and the titrant; receiving the color parameter detected by the color sensor 6 during the titration; and controlling the waste removal device 8 to discharge the waste. The control unit 9 calculates the content of the calcium ion in the test solution according to the volume of the test solution and the volume of the titrant detected by the first light interception counter 7 and further calculates the content of the calcium ion of the liquid of the seawater aquarium fish tank.

The supplementary device 10 includes a supplementary suction tube 101, a supplement storage tank 102, a first supplementary pump 103, a supplement tank 104, a second supplementary pump 105, a supplement output tube 106, and a second light interception counter 107. One end of the supplementary suction tube 101 extends into the supplement storage tank 104, another end of the supplementary suction tube has a dropper tip 1011 and aiming an opening 1041 of the supplement tank 104. The first supplement pump 103 drives the supplementary suction tube 101 to draw the supplement 1021 in the supplement storage tank 102 and drop the supplement 1021 into the supplement tank 104 through the dropper tip 1011 of the supplementary suction tube 101. The second light interception counter 107 counts the number of drop of the supplement 1021 for controlling the volume of the supplement 1021 that drops into the supplement tank 104. The supplementary output tube interconnects with the supplement tank 104. The second supplementary pump 105 drives the supplementary output tube 106 to draw the supplement 1021 out from the supplement tank 104 and discharge the supplement 1021 into the seawater aquarium fish tank to maintain the content of the calcium ion in the water of the seawater aquarium fish tank at a predetermined value.

The method for operating the automatic water quality adjustment system of the present embodiment is described below, please refer to FIG. 4, the method comprises the steps of: Step (1): the sample pump 23 of the sample dripping device 2 drove the sample suction tube 21 to draw a portion of the water of the aquarium fish tank as a test solution, and drove the sample dropper 22 to drop 5 mL of the test solution (500 drops, counted by the first light interception counter 7) into the reaction tank 1. Step (2): the sample pump 23 provided the power to draw the remaining test solution in the sample suction tube 21 and the sample dropper 22 back to the aquarium fish tank. Step (3): the precipitant pump 34 of the precipitant dripping device 3 drove the precipitant suction tube 31 to draw the precipitant 331 from the precipitant storage tank 33, and drove the precipitant dropper 32 to drop 25 drops of the precipitant 331 (counted by the first light interception counter 7) into the reaction tank 1 through the precipitant dropper 32, at the meantime, the precipitant 331 and the test solution was uniformly mixed by the stirrer 20. Step (4): the precipitant pump 34 provides the power to draw the remaining precipitant 331 in the precipitant suction tube 31 and the precipitant dropper 32 back to the precipitant storage tank 33. Step (5): the indicator pump 44 of the indicator dripping device 4 drove the indicator suction tube 41 to draw the indicator 431 from the indicator storage tank 43 and drove the indicator dropper 42 to drop 14 drops (counted by the first light interception counter 7) of the indicator 431 into the reaction tank 1, at the meantime, the indicator 431 and the test solution was uniformly mixed by the stirrer 20. Step (6): the indicator pump 44 provided power to draw the remaining indicator 431 in the indicator suction tube 41 and the indicator dropper 42 back to the indicator storage tank 43. Step (7): the titrant pump 54 of the titrant dripping device 5 drove the titrant suction tube 51 to draw the titrant 531 from the titrant storage tank 53, and drove the titrant dropper 52 to drop 60 drops of the titrant 531 into the reaction tank 1, at the meantime, the titrant 531 and the test solution was uniformly mixed by the stirrer 20. Step (8): one drop of the titrant 531 was dropped into the reaction tank 1 in every three seconds, and the stirrer 20 stop stirring three seconds after dropping one drop of the titrant 531, and then the color sensor 6 detected the color of the test solution, wherein the number of drops of the titrant 531 was counted by the first light interception counter 7. Step (9): stop dropping the titrant 531 when the color sensor 6 detected the color of the test solution became blue. Step (10): the titrant pump 54 provided power to draw the remaining titrant 531 of the titrant suction tube 51 and the titrant dropper 52. Step (11): the waste pump 83 of the waste removal device 8 drove the waste suction tube 81 to draw the waste from the reaction tank 1 and drove the waste discharge tube 82 to discharge the waste to the waste storage tank 84. Step (12) the sample pump 23 of the sample dripping device 2 drove the sample suction tube 21 to draw 7 mL of water from the aquarium fish tank and drove the sample dropper 22 to drop 500 drops of the water into the reaction tank 1. The water was stirred by the stirrer for 10 seconds, and then the waste pump 83 drove the waste suction tube 81 to draw the waste in the reaction tank 1 and discharge the waste to the waste storage tank 84 for washing the reaction tank 1. Step (13): the control unit 9 calculated the content of calcium ion in the aquarium fish tank according to the titter of titrating the test solution, further, the control unit 9 calculated the amount of calcium ion that should be added into the aquarium fish tank. Step (14): The first supplementary pump 103 of the supplementary device 10 to drove the supplementary suction tube 101 to draw the supplement 1021 from the supplement storage tank 102, and drove the supplementary suction tube 101 to drop the supplement 1023 into the supplement tank 104 through the dropper tip 1011 until the supplement tank 104 was filled with sufficient supplement, wherein the number of drop of the supplement was counted by the second light interception counter 107. Step (15): The second supplementary pump 105 drove the supplement discharge tube 106 to draw the supplement 1021 in the supplement tank 104 and to discharge the supplement 1021 to the aquarium fish tank.

The present embodiment is exemplified by adjusting the content of calcium ions in a seawater aquarium fish tank. However, instead of adjusting the content of calcium ion in the water of the fish tank, the content of magnesium ion and the water hardness are also critical to maintain the healthiness of the aquatic creatures in the fish tank. Accordingly, in other embodiment of the present invention, the number of the precipitant dripping device, the indicator dripping device, the titrant dripping device, and the supplementary device are not particularly limited and can be adjusted based on the needs. For example, if the automatic water quality adjustment system is designed to adjust the content of calcium ion, the content of magnesium ion, and the hardness of water (Kh), the automatic water quality adjustment system will comprise two precipitant dripping devices (a precipitant is not needed when detecting the hardness of water), three indicator dripping devices, three titrant dripping devices, and three supplementary devices.

Although the present invention has been explained in relation to its preferred embodiment, it is to be understood that many other possible modifications and variations can be made without departing from the scope of the invention as hereinafter claimed.

## Claims

1. An automatic water quality adjustment system for detecting a liquid, comprising:
a reaction tank including an opening;
a sample dripping device including a sample suction tube and a sample dropper, a dropper tip of the sample dropper extends to the opening of the reaction tank and interconnects with the sample suction tube, so that a test solution, which is a portion of the liquid drawn by the sample suction tube, is dropped into the reaction tank;
at least one titrant dripping device including a titrant dropper, a dropper tip of the titrant dropper extends to the opening of the reaction tank so that a titrant is dropped into the reaction tank for titrating the test solution in the reaction tank;
a color sensor disposed on a side of the reaction tank for detecting the color change of the test solution when reacting with the titrant;
a first light interception counter disposed adjacent to the opening for counting number of drops of the test solution and the titrant dropped into the reaction tank;
a waste removal device including a waste suction tube and a waste discharge tube, one end of the waste suction tube interconnects with the reaction tank, another end of the waste suction tube interconnects with the waste discharge tube, wherein the waste discharge tube draws the waste liquid generated after completion of the titration in the reaction tank;
a control unit controlling drop frequency and number of drops of the test solution and the titrant; discharging waste of the waste removal device; and receiving and processing signals from the color sensor and the first light interception counter; and
a supplementary device including a supplement output tube, one end of the at least one supplement output tube output at least one supplement to the liquid;
wherein the at least one titrant dripping device titrates the test solution in the reaction tank with the titrant; the first light interception counter counts the number of drops of the titrant; the color sensor detects the color change of the test solution and reacting with the titrant to determine whether the titration is complete; and the control unit calculates a titer of the titrant detected by the first light interception counter and processes the titer of the titrant into an output amount of the supplement; and the supplement of the output amount is outputted to the liquid.

2. The automatic water quality adjustment system as claimed in claim 1, further comprising at least one precipitant dripping device, wherein the at least one precipitant dripping device includes a precipitant dropper, a dropper tip of the precipitant dropper extends to the opening of the reaction tank so that a precipitant is dropped into the reaction tank for precipitating impurities of the test solution in the reaction tank.

3. The automatic water quality adjustment system as claimed in claim 1 or 2, further comprising at least one indicator dripping device, wherein the at least one indicator dripping device includes an indicator dropper, a dropper tip of the indicator dropper extends to the opening of the reaction tank so that an indicator is dropped into the reaction tank for reacting with the test solution and rendering color.

4. The automatic water quality adjustment system as claimed in claim 2, wherein the at least one precipitant dripping device further includes a precipitant suction tube, a precipitant storage tank, and a precipitant pump, the precipitant is stored in the precipitant storage tank, one end of the precipitant suction tube extends to the precipitant storage tank, another end of the precipitant suction tube connects with the precipitant pump, and a tube body of the precipitant dropper also connects with the precipitant pump, wherein the precipitant pump drives the precipitant suction tube to draw the precipitant from the precipitant storage tank and drives the precipitant dropper to drop the precipitant into the reaction tank.

5. The automatic water quality adjustment system as claimed in claim 3, wherein the at least one indicator dripping device further includes an indicator suction tube, an indicator storage tank, and an indicator pump, the indicator is stored in the indicator storage tank, one end of the indicator suction tube extends to the indicator storage tank, another end of the indicator suction tube connects with the indicator pump, and a tube body of the indicator dropper also connects with the indicator pump, wherein the indicator pump drives the indicator suction tube to draw the indicator from the indicator storage tank and drives the indicator dropper to drop the indicator into the reaction tank.

6. The automatic water quality adjustment system as claimed in any of the previous claims, wherein the sample dripping device further includes a sample pump, one end of the sample suction tube extends into the liquid, another end of the sample suction tube connects with the sample pump, and a tube body of the sample dropper also connects with the sample pump, wherein the sample pump drives the sample suction tube to draw the portion of the liquid as the test sample and drives the sample dropper to drop the test sample into the reaction tank.

7. The automatic water quality adjustment system as claimed in any of the previous claims, wherein the at least one titrant dripping device further includes a titrant suction tube, a titrant storage tank, and a titrant pump, the titrant is stored in the titrant storage tank, one end of the titrant suction tube extends to the titrant storage tank, another end of the titrant suction tube connects with the titrant pump, and a tube body of the titrant dropper also connects with the titrant pump, wherein the titrant pump drives the titrant suction tube to draw the titrant from the titrant storage tank and drives the titrant dropper to drop the titrant into the reaction tank.

8. The automatic water quality adjustment system as claimed in any of the previous claims, wherein the waste removal device further includes a waste pump, wherein another end of the waste suction tube connects with the waste pump, wherein the waste pump drives the waste suction tube to draw the waste from the reaction tank and discharges the waste from the waste discharge tube.

9. The automatic water quality adjustment system as claimed in any of the previous claims, wherein the supplementary device further includes at least one supplementary suction tube, at least one supplement storage tank, at least one supplementary pump, a supplement tank, and a second supplementary pump, wherein one end of the at least one supplementary suction tube extends to the at least one supplement storage tank, a dropper tip at another end of the at least one supplementary suction tube extends to an opening of the supplement tank, at least one supplement is stored in the at least one supplement storage tank respectively, and the supplementary output tube interconnects with the supplement tank, wherein the at least one first supplementary pump drives the at least one supplementary suction tube to draw the at least one supplement from the at least one supplement storage tank and drop the at least one supplement into the supplement tank, then the second supplementary pump drives the supplementary output tube to output the at least one supplement into the liquid.

10. The automatic water quality adjustment system as claimed in claim 9, wherein the supplementary device further includes a second light interception counter disposed at the opening of the supplement tank for counting the number of drops of the at least one supplement dropped into the supplement tank.

11. The automatic water quality adjustment system as claimed in any of the previous claims, further comprising a stirrer disposed in the reaction tank for uniformly mixing the test solution and at least one selected from the group consisting of the precipitant, the indicator, and the titrant.

12. An automatic water quality adjustment method using the automatic water quality adjustment device claimed in any one of claims 1 to 3, comprising the steps of:
Step (1): using the sample dripping device to draw a portion of the liquid as the test solution and to drop the test solution into the reaction tank, and using the first light interception counter to count the number of drops of the test solution for calculating a volume of the test solution;
Step (2): using the at least one titrant dripping device to drop the titrant into the reaction tank to react with the test solution for performing a titration, using the first light interception counter to record the number of drops of the titrant, and using the color sensor to detect the color change while titration, when the color of the test solution reaches a pre-determined color, the titration is completed; and
Step (3): using the control unit to calculate the titer of the titrant detected by the first interception counter and process the titer of the titrant into the output amount of the supplement, then the supplement with the output amount is outputted to the liquid by the supplementary device.

13. The automatic water quality adjustment method as claimed in claim 12, further comprising a Step (1-1): using the at least one precipitant dripping device to drop the precipitant into the reaction tank for reacting the precipitant and the test solution.

14. The automatic water quality adjustment method as claimed in claim 12 or 13, further comprising a Step (1-2): using at least one the indicator dripping device to drop the indicator into the reaction tank for reacting the indicator and the test solution.

15. The automatic water quality adjustment method as claimed in claim 12, 13 or 14, further comprising a Step (2-1): using the waste removal device to discharge the waste in the reaction tank after the Step (2).
